(19) Europäisches Patentamt
European Patent Office
Office européen des brevets

(11) Veröffentlichungsnummer: **0 005 471**
**B1**

(12) # EUROPÄISCHE PATENTSCHRIFT

(45) Veröffentlichungstag der Patentschrift: **09.09.81**

(51) Int. Cl.$^3$: **C 07 C 67/44**, C 07 C 69/28

(21) Anmeldenummer: **79101285.9**

(22) Anmeldetag: **30.04.79**

(54) **Verfahren zur Herstellung von 3-Hydroxy-2,2,4-trimethylpentylisobutyrat.**

(30) Priorität: **11.05.78 DE 2820518**

(43) Veröffentlichungstag der Anmeldung:
**28.11.79 Patentblatt 79/24**

(45) Bekanntmachung des Hinweises auf die Patenterteilung:
**09.09.81 Patentblatt 81/36**

(84) Benannte Vertragsstaaten:
**BE DE FR GB NL**

(56) Entgegenhaltungen:
**DE - B - 1 183 899**

(73) Patentinhaber: **BASF Aktiengesellschaft**
**Carl-Bosch-Strasse 38**
**D-6700 Ludwigshafen (DE)**

(72) Erfinder: **Merger, Franz, Dr.**
**Max-Slevogt-Strasse 25**
**D-6710 Frankenthal (DE)**
Erfinder: **Foerster, Hans-Juergen, Dr.**
**Leipziger Strasse 10**
**D-6712 Bobenheim-Roxheim 2 (DE)**

**EP 0 005 471 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von 3-Hydroxy-2,2,4-trimethylpentylisobutyrat

Die Erfindung betrifft ein neues Verfahren zur Herstellung von 3-Hydroxy-2,2,4-trimethylpentylisobutyrat durch Umsetzung von Isobutyraldehyd in Gegenwart von Erdalkalihydroxid unter Zusatz von Carbonsäuren bzw. entsprechenden Salzen, unter bestimmten Bedingungen bezüglich Reaktionszeit, Mengenverhältnis und Konzentration der Hydroxidsuspensionen.

Es ist aus den Monatsheften der Chemie, Band 25, Seiten 188 bis 196, bekannt, daß man Isobutyraldehyd in Gegenwart von Kalkwasser oder Kalkmilch zu 3-Hydroxy-2,2,4-trimethylpentylisobutyrat umsetzt. Die Ausbeuten sind jedoch sehr schlecht und es entstehen eine Reihe unerwünschter Nebenprodukte wie Isobutyraldol, Isobutyraldoxan, durch Verseifung gebildetes 2,2,4-Trimethylpentandiol-1,3 sowie Isobutylisobutyrat.

In der deutschen Patentschrift 646 482 wird die Herstellung von 1,3-Glykolhalbestern durch Kondensation entsprechender Aldehyde mit nur einem zur Carbonylgruppe $\alpha$-ständigem Wasserstoffatom, z.B. 2-Methylpropanol, 2-Methylbutanal, 2-Methylpentanal, in Abwesenheit von Lösungsmitteln mit festem Natriumhydroxid oder Natriumamid als Katalysator beschrieben. Die Aufarbeitung durch Ansäuern mit Salzsäure, Neutralwaschen mit Wasser und sich daran anschließende Wasserdampfdestillation und Destillation ist relativ aufwendig. Wie schon die US-Patentschrift 3 718 689 festellt (Spalte 1, Zeilen 10 bis 22), sind die Nachteile im Zusammenhang mit der Verwendung von festem Alkali in chemischen Prozessen offensichtlich (schwierige Dosierung und Auflösung des Katalysators, schlechte Regelung des Betriebs), festes NaOH zerfließt an der Luft und bedeutet eine Gefahr für das damit arbeitende Personal. Es wird zum Stande der Technik in DBP 646 482 ausdrücklich (Seite 1, Zeilen 27 bis 31 und 58 bis 60) angegeben, daß Natriumacetat und Kalkmilch als Katalysatoren einer Herstellung von 3-Hydroxy-2,2,4-trimethylpentylisobutyrat ungeeignet sind, da sie sehr schlechte Ausbeuten an Endstoff liefern und die vorgenannten Nebenprodukte gebildet werden.

Die US-Patentschrift 3 291 821 und die US-Patentschrift 3 718 689 beschreiben kontinuierliche Verfahren mit wäßriger Natronlauge als Katalysator, beim ersten Verfahren mit 5- bis 20-gewichtsprozentiger Natronlauge, beim zweiten Verfahren mit 30- bis 50-gewichtsprozentiger Natronlauge. Die Ausbeute ist bei beiden Verfahren im allgemeinen unbefriedigend und bei dem Verfahren der US-Patentschrift 3 718 689 im laufenden Betrieb erheblich schwankend. Zwar geben beiden US-Patentschriften an, daß auch Erdalkalihydroxide als Katalysatoren verwendet werden können, doch kommen nur Alkalilaugen in den Beispielen zur Anwendung. Gleichzeitig wird in der US-Patentschrift 3 718 689 beansprucht und näher beschrieben, daß die wäßrigen Lösungen der Hydroxide mindestens eine Konzentration von 30 Gewichtsprozent Hydroxid, bezogen auf das Gesamtgewicht der wäßrigen Lösung, aufweisen müssen. Diese Bedingung zeigt, daß praktisch das Verfahren nur mit Alkalihydroxidlösungen durchgeführt werden kann, da ja Erdalkali nur in Gestalt einer wäßrigen Suspension und nicht einer wäßrigen Lösung in einer Konzentration von mindestens 30 Gewichtsprozent vorliegen kann. Eine Säure wird dem Ausgangsgemisch nicht zugesetzt. Der Säureanteil des Ausgangsaldehyds sollte, wie die US-Patentschrift 3 718 689, Spalte 3, Zeilen 10 bis 15 zeigen, 0,5 Gewichtsprozent, bezogen auf den Aldehyd, nicht übersteigen; in den Beispielen beträgt der Säureanteil 0,36 Gewichtsprozent. Die Patentschrift 3 718 689 weist darauf hin (Spalte 4, Zeilen 21 bis 31), daß zur Erzielung optimaler Raum-Zeit-Ausbeuten eine Verweilzeit von höchstens 10 Minuten eingehalten werden sollte und zur Aufarbeitung des Reaktionsgemisches zweckmäßig eine Wasserdampfdestillation bei 125°C und 2 at Druck durchgeführt wird, um das als Nebenprodukt anfallende Isobutyraldoxan (Trimer) zu spalten und den so gebildeten Aldehyd (Monomer) als azeotropes Gemisch mit Wasser abzutrennen. Eine ähnliche azeotrope Abtrennung empfiehlt auch die US-Patentschrift 3 291 821.

In der US-Patentschrift 3 703 541 ist ein Verfahren beschrieben, das einen Umsatz von 84,3 Prozent mit einer Ausbeute an 3-Hydroxy-2,2,4-trimethylpentylisobutyrat von 92 Prozent erreicht, die Art der Katalyse mit Alkaliphenolaten in z.B. Toluol als Lösungsmittel ist aber umständlich, nicht störungssicher und teuer. Außerdem wird darauf hingewiesen, daß ein steigender Wassergehalt den Umsatz und die Selektivität verringern, und daher ein Gehalt von weniger als 5 500 ppm gefordert wird. In den Beispielen liegt der Wassergehalt meist bei ca. 1 000 ppm. Die so für das Verfahren notwendige technische Entwässerung von Isobutyraldehyd ist aufwendig.

Es wurde nun gefunden, daß man 3-Hydroxy-2,2,4-trimethylpentylisobutyrat durch Umsetzung von Isobutyraldehyd in Gegenwart von basischen Verbindungen und Wasser vorteilhaft erhält, wenn man Isobutyraldehyd unter Zusatz von 1,5 bis 5 Gewichtsprozent Carbonsäure, bezogen auf Isobutyraldehyd, in Gestalt der freien Säure, ihres Alkalisalzes und/oder ihres Erdalkalisalzes, und in Gegenwart von Erdalkalihydroxiden und Wasser in einer Menge von 0,01 bis 0,1 Mol Erdalkalihydroxid und von 0,04 bis 1 Mol Wasser je Mol Isobutyraldehyd, während einer Reaktionszeit zwischen 10 und 300 Minuten umsetzt.

Die Umsetzung kann durch die folgenden Formeln wiedergegeben werden:

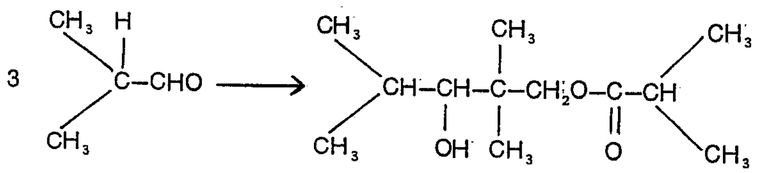

Das Verfahren nach der Erfindung geht von der Beobachtung aus, daß nur eine ganz spezielle Kombination ausgewählter Merkmale, die bisher noch nie in der erfindungsgemäßen Kombination als zweckmäßig erkannt wurden, optimale Ergebnisse erzielen lassen. Im Vergleich zu den bekannten Verfahren liefert es auf einfacherem und wirtschaftlichem Wege 3-Hydroxy-2,2,4-trimethylpentylisobutyrat in besserer Ausbeute und Reinheit. Weit weniger Isobutyraldol und Isobutyraldoxan fallen an. Andererseits katalysieren die beanspruchten Katalysatoren die Folgereaktionen von 3-Hydroxy-2,2,4-trimethylpenylisobutyrat zu 2,2,4-Trimethylpentandiol-1,3 und dem Diisobutyrat des 2,2,4-Trimethylpentandiol-1,3 erheblich schwächer:

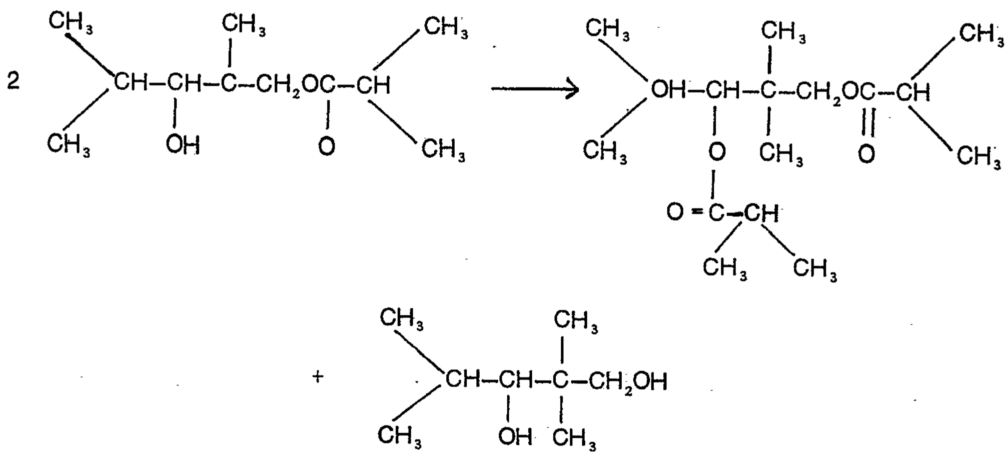

Man erhält hohe Ausbeuten an Endstoff in leichter isolierbarer Form. Ein beträchtlicher Vorteil ist die erfindungsgemäße Verwendbarkeit von Wasser, da Wasser enthaltender Isobutyraldehyd technisch leichter zugänglich ist als wasserfreier. Sorgfältiger Sauerstoffausschluß ist nicht nötig, es ist sogar möglich, die Säure in situ durch Ein- oder Überleiten von sauerstoffhaltigen Gasen zu erzeugen. Die Reaktion verläuft in Gegenwart der erfindungsgemäßen Carbonsäuren oder carebonsauren Salzen erheblich schneller als mit den Erdalkalihydroxiden allein, sowohl selektiver als auch mit höherer Umwandlung im Vergleich zu den bisher bekannten Verfahren. Aufwendige Aufarbeitungsschritte, z.B. Wasserdampfdestillation, sind nicht notwendig, Alle diese vorteilhaften Ergebnisse sind im Hinblick auf den Stand der Technik überraschend.

Der Isobutyraldehyd kann in der Regel bis zur Löslichkeitsgrenze, vorzugsweise von 0 bis 5 Gewichtsprozent Wasser, bezogen auf Isobutyraldehyd, enthalten. Dieser Wassergehalt ist ebenfalls in der erfindungsgemäßen Konzentration an Wasser enthalten. Die Umsetzung wird im allgemeinen bei einer Temperatur von 20 bis 150, vorzugsweise von 40 bis 90°C, drucklos oder unter Druck, z.B. bei 0,98 bis 10 bar, kontinuierlich oder diskontinuierlich durchgeführt. Man verwendet vorzugsweise Bariumhydroxid, Strontiumhydroxid und/oder insbesondere Calciumhydroxid als Erdalkalihydroxide, zweckmäßig in Mengen von 0,01 bis 0,1, vorzugsweise von 0,015 bis 0,07 Mol Erdalkalihydroxid je Mol Isobutyraldehyd. Das Erdalkalihydroxid befindet sich während der Reaktion nicht in wäßriger Lösung, sondern im allgemeinen in wäßriger Suspension oder im Einzelfall auch in Form einer feuchten Reaktionsmasse. Die Konzentration an Wasser beträgt von 0,04 bis 1, vorzugsweise 0,08 bis 0,5, insbesondere 0,16 bis 0,3 Mol Wasser je Mol Isobutyraldehyd. Im allgemeinen werden außer Wasser keine weiteren Lösungsmittel verwendet. Die Reaktionszeit beträgt zwischen 10 und 300, vorzugsweise von 20 bis 200, insbesondere 30 bis 180 Minuten.

Als Carbonsäuren können solche mit mehreren, zweckmäßig 2 Carboxylgruppen, und bevorzugt mit einer Carboxylgruppe verwendet werden, wobei Carbonsäuren mit mindestens 3 Kohlenstoffatomen besonders bevorzugt sind. Vorteilhaft werden sie in Gestalt ihrer Magnesiumsalze, Lithiumsalze, Kaliumsalze, Insbesondere Bariumsalze, Strontiumsalze und bevorzugt ihrer Calciumsalze, Natriumsalze oder als freie Säuren, angewendet. Man setzt 1,5 bis 5, zweckmäßig 1,5 bis 3, vorzugsweise 1,5 bis 2 Gewichtsprozent Carbonsäuren, bezogen auf Isobutyraldehyd, zu, wobei die Carbonsäuren in Gestalt entweder der freien Säure oder des Alkalisalzes oder des Erdalkalisalzes oder in Gestalt eines Gemisches der Säure und ihres Alkalisalzes oder in Gestalt eines Gemisches der Säure und ihres Erdalkalisalzes oder in Gestalt eines Gemisches des Alkalisalzes und des Erdalkalisalzes einer Carbonsäure oder in Gestalt eines Gemisches der Carbonsäure und ihres Alkalisalzes und ihres Erdalkalisalzes verwendet wird. Die vorgenannten Gewichtsprozentangaben verstehen sich berechnet als freie 100-prozentige Carbonsäure, ohne Berücksichtigung der wirklich verwendeten Carbonsäureverbindung. Es können gegebenenfalls auch Gemische verwendet werden, die mehrere Carbonsäuren, carbonsaure Alkalisalze und/oder Erdalkalisalze enthalten; ebenfalls kommen polybasische, nur teilweise durch Alkali und/oder Erdalkali neutralisierte Carbonsäuren in Betracht.

Beispielsweise sind folgende Säuren geeignet: aliphatische Carbonsäuren, insbesondere Alkancarbonsäuren mit 3 bis 10 Kohlenstoffatomen, wie Propionsäure, Buttersäure, Isobuttersäure, Capronsäure, Pelargonsäure, $\beta$-Isobutyroxypivalinsäure, $\alpha$-Methylpentansäure, 3,5,5-Trimethylhexansäure, 2,2,4-Trimethyl-3-hydroxy-pentansäure, 2-Äthylpenten-(2)-säure-(1), 2-Äthylhexancarbonsäure, $\alpha$-Äthylbuttersäure, Isovaleriansäure, Valeriansäure; cycloaliphatische Carbonsare, insbesondere Cycloalkylcarbonsäuren mit 6 bis 8 Kohlenstoffatomen, araliphatische Carbonsäuren, insbesondere mit 8 bis 12 Kohlenstoffatomen, aromatische Carbonsäuren, insbesondere mit 7 bis 12 Kohlenstoffatomen, wie Benzoesäure, Phenylpropionsäure, Phenylessigsäure oder entsprechende Gemische; entsprechende Natrium- oder Calciumsalze. Bevorzugt sind Isobuttersäure, 2,2,4-Trimethjyl-3-hydroxy-pentansäure, 2-Äthylhexancarbonsäure, Benzoesäure, Phenylpropionsäure, $\alpha$-Methylpentancarbonsäure.

Die Reaktion kann wie folgt durchgeführt werden: Ein Gemisch von Isobutyraldehyd, Erdalkalihydroxid, Wasser und Carbonsäure wird während der Reaktionszeit bei der Reaktionstemperatur gehalten. Man kann z.B. dem Isobutyraldehyd den gewünschten Gehalt an Wasser und Säure zusetzen und den Katalysator im Gemisch suspendieren. Dann wird der Endstoff aus dem Reaktionsgemisch in üblicher Weise, z.B. durch Neutralisation und Destillation des Gemisches, abgetrennt.

Das nach dem Verfahren der Erfindung hergestellte 3-Hydroxy-2,2,4-trimethylpentylisobutyrat ist ein wertvoller Ausgangsstoff für die Herstellung von Farbstoffen, Schädlingsbekämpfungsmitteln, Klebstoffen und Lacken. Es ist ein ausgezeichnetes Verlaufshilfsmittel für Klebstoffe und Lacke auf Polyvinylacetat- und Polyacrylat-Basis. Weiterhin findet es als Lösungsmittel und Weichmacher in Latexemulsionen Verwendung. Seine Ester dienen zum Plastifizieren von plastischen Polymeren. Bezüglich der Verwendung wird auf vorgenannte Veröffentlichungen und die deutsche Patentschrift 1 291 041 verwiesen.

Die in den folgenden Beispielen aufgeführten Teile bedeuten Gewichtsteile.

## Beispiel 1

900 Teile Isobutyraldehyd, 54 Teile $Ca(OH)_2$ und 54 Teile Wasser und 18 Teile Isobuttersäure werden während 2 Stunden bei 60 bis 80°C unter Stickstoff am Rückfluß erhitzt. Man erhält (gaschromatographische Analyse) 711,9 Teile 3-Hydroxy-2,2,4-trimethylpentylisobutyrat (79,1% der Theorie), 11,7 Teile 2,2,4-Trimethylpentandiol-1,3 und 166,5 Teile unumgesetzten Ausgangsstoff. Das Reaktionsgemisch wird dann mit 34 Teilen Kohlendioxid neutralisiert und der Endstoff durch Destillation abgetrennt. 3-Hydroxy-2,2,4-trimethylpentylisobutyrat hat einen Kp von 128°C/20 mbar.

## Beispiel 2

250 Teile Isobutyraldehyd, 15 Teile $Ca(OH)_2$ und 15 Teile Wasser und 5 Teile Isobuttersäure werden während 2 Stunden bei 60 bis 80°C in Anwesenheit von Luft am Rückfluß erhitzt. Man erhält (gaschromatographische Analyse) 188,8 Teile 3-Hydroxy-2,2,4-trimethylpentylisobutyrat (75,5% der Theorie), 4,4 Teile 2,2,4-Trimethylpentandiol-1,3 und 52,8 Teile unumgesetzen Ausgangsstoff. Das Reaktionsgemisch wird dann mit 10 Teilen Kohlendioxid neutralisiert und der Endstoff durch Destillation abgetrennt. 3-Hydroxy-2,2,4-trimethylpentylisobutyrat hat einen Kp von 128°C/20 mbar.

## Beispiel 3

250 Teile Isobutyraldehyd, 15 Teile $Ca(OH)_2$ und 15 Teile Wasser und 5 Teile Calciumisobutyrat werden während 2 Stunden bei 60 bis 74°C in Anwesenheit von Luft am Rückfluß erhitzt. Man erhält (gaschromatographische Analyse) 153,3 Teile 3-Hydroxy-2,2,4-trimethylpentylisobutyrat (61,3% der Theorie), 0,7 Teile 2,2,4-Trimethylpentandiol-1,3 und 92,5 Teile unumgesetzten Ausgangsstoff. Das Reaktionsgemisch wird dann mit 10 Teilen Kohlendioxid neutralisiert und der Endstoff durch Destillation abgetrennt. 3-Hydroxy-2,2,4-trimethylpentylisobutyrat hat einen Kp von 128°C/20 mbar.

## Beispiel 4

250 Teile Isobutyraldehyd, 5 Teile $Ca(OH)_2$ und 15 Teile Wasser und 5 Teile Isobuttersäure werden während 3 Stunden bei 60 bis 80°C in Anwesenheit von Luft am Rückfluß erhitzt. Man erhält (gaschromatographische Analyse) 191,3 Teile 3-Hydroxy-2,2,4-trimethylpentylisobutyrat (76,5% der Theorie), 4 Teile 2,2,4-Trimethylpentandiol-1,3 und 51 Teile unumgesetzten Ausgangsstoff. Das Reaktionsgemisch wird dann mit 10 Teilen Kohlendioxid neutralisiert und der Endstoff durch Destillation abgetrennt. 3-Hydroxy-2,2,4-trimethylpentylisobutyrat hat einen Kp von 128°C/20 mbar.

Beispiel 5

250 Teile Isobutyraldehyd, 15 Teile $Ca(OH)_2$ und 15 Teile Wasser und 8,2 Teile 2-Äthylhexansäure werden während 2 Stunden bei 60 bis 81°C in Anwesenheit von Luft am Rückfluß erhitzt. Man erhält (gaschromatographische Analyse) 189,5 Teile 3-Hydroxy-2,2,4-trimethylpentylisobutyrat (75,8% der Theorie), 8,8 Teile 2,2,4-Trimethylpentandiol-1,3 und 42,5 Teile unumgesetzten Ausgangsstoff. Das Reaktionsgemisch wird dann mit 10 Teilen Kohlendioxid neutralisiert und der Endstoff durch Destillation abgetrennt. 3-Hydroxy-2,2,4-trimethylpentylisobutyrat hat einen Kp von 128°C/20 mbar.

Beispiel 6

250 Teile Isobutyraldehyd, 15 Teile $Ca(OH)_2$ und 15 Teile Wasser und 8,75 Teile Trimethyl-3-hydroxy-pentansäure werden während 1,75 Stunden bei 60 bis 80°C in Anwesenheit von Luft am Rückfluß erhitzt. Man erhält (gaschromatographische Analyse) 191,5 Teile 3-Hydroxy-2,2,4-trimethylpentylisobutyrat (76,6% der Theorie), 9 Teile 2,2,4-Trimethylpentandiol-1,3 und 37,8 Teile unumgesetzen Ausgangsstoff. Das Reaktionsgemisch wird dann mit 10 Teilen Kohlendioxid neutralisiert und der Endstoff durch Destillation abgetrennt. 3-Hydroxy-2,2,4-trimethylpentylisobutyrat hat einen Kp von 128°C/20 mbar.

Beispiel 7

250 Teile Isobutyraldehyd, 15 Teile $Ca(OH)_2$ und 15 Teile Wasser und 5 Teile Natriumbenzoat werden während 4 Stunden bei 60 bis 80°C in Anwesenheit von Luft am Rückfluß erhitzt. Man erhält (gaschromatographische Analyse) 200 Teile 3-Hydroxy-2,2,4-trimethylpentylisobutyrat (80% der Theorie), 10,3 Teile 2,2,4-Trimethylpentandiol-1,3 und 32,5 Teile unumgesetzen Ausgangsstoff. Das Reaktionsgemisch wird dann mit 10 Teilen Kohlendioxid neutralisiert und der Endstoff durch Destillation abgetrennt. 3-Hydroxy-2,2,4-trimethylpentylisobutyrat hat einen Kp von 128°C/20 mbar.

Beispiel 8

250 Teile Isobutyraldehyd, 15 Teile $Ca(OH)_2$ und 15 Teile Wasser und 5 Teile 2-Phenylpropionsäure werden während 2 Stunden bei 60 bis 77°C in Anwesenheit von Luft am Rückfluß erhitzt. Man erhält (gaschromatographische Analyse) 175,5 Teile 3-Hydroxy-2,2,4-trimethylpentylisobutyrat (70,2% der Theorie), 6,8 Teile 2,2,4-Trimethylpentandiol-1,3 und 63,8 Teile unumgesetzten Ausgangsstoff. Das Reaktionsgemisch wird dann mit 10 Teilen Kohlendioxid neutralisiert und der Endstoff durch Destillation abgetrennt. 3-Hydroxy-2,2,4-trimethylpentylisobutyrat hat einen Kp von 128°C/20 mbar.

Beispiel 9

150 Teile isobutyraldehyd, 9 Teile $Ba(OH)_2$ und 9 Teile Wasser und 3 Teile Isobuttersäure werden während 0,25 Stunden bei 60 bis 80°C in Anwesenheit von Luft am Rückfluß erhitzt. Man erhält (gaschromatographische Analyse) 107,3 Teile 3-Hydroxy-2,2,4-trimethylpentylisobutyrat (71,5% der Theorie), 8,7 Teile 2,2,4-Trimethylpentandiol-1,3 und 30,6 Teile unumgesetzten Ausgangsstoff. Das Reaktionsgemisch wird dann mit 6 Teilen Kohlendioxid neutralisiert und der Endstoff durch Destillation abgetrennt. 3-Hydroxy-2,2,4-trimethylpentylisobutyrat hat einen Kp von 128°C/20 mbar.

Vergleich

Die Umsetzung wird analog Beispiel 1, aber ohne Zusatz von Isobuttersäure durchgeführt. Man erhält (gaschromatographische Analyse) 241,2 Teile 3-Hydroxy-2,2,4-trimethylpentylisobutyrat (26,8% der Theorie), 5 Teile 2,2,4-Trimethylpentandiol-1,3 und 648 Teile unumgesetzten Ausgangsstoff. Das Reaktionsgemisch wird dann mit 34 Teilen Kohlendioxid neutralisiert und der Endstoff durch Destillation abgetrennt. 3-Hydroxy-2,2,4-trimethylpentylisobutyrat hat einen Kp von 128°C/20 mbar.

**Patentansprüch**

Verfahren zur Herstellung von 3-Hydroxy-2,2,4-trimethylpentylisobutyrat durch Umsetzung von Isobutyraldehyd in Gegenwart von basischen Verbindungen und Wasser, dadurch gekennzeichnet, daß man Isobutyraldehyd unter Zusatz von 1,5 bis 5 Gewichtsprozent Carbonsäure, bezogen auf Isobutyraldehyd, in Gestalt entweder der freien Säure oder des Alkalisalzes oder des Erdalkalisalzes oder in Gestalt eines Gemisches der Säure und ihres Erdalkalisalzes oder in Gestalt eines Gemisches der Säure und ihres Alkalisalzes oder in Gestalt eines Gemisches des Alkalisalzes und des Erdalkalisalzes einer Carbonsäure oder in Gestalt eines Gemisches der Carbonsäure und ihres Alkalisalzes und ihres Erdalkalisalzes, und in Gegenwart von Erdalkalihydroxiden und Wasser in einer Menge von 0,01 bis 0,1 Mol Erdalkalihydroxid und von 0,04 bis 1 Mol Wasser je Mol Isobutyraldehyd, während einer Reaktionszeit zwischen 10 und 300 Minuten unsetzt.

## Revendication

Procédé pour la préparation d'isobutyrate 3-hydroxy-2,2,4-triméthylpentylique par transformation d'aldéhyde isobutyrique en présence de composés basiques et d'eau, caractérisé en ce qu'on fait réagir l'aldéhyde isobutyrique pendant une durée de réaction comprise entre 10 et 300 minutes, avec addition de 1,5 à 5% en poids d'acide carboxylique, par rapport à l'aldéhyde isobutyrique, soit sous forme de l'acide libre, de son sel alcalin ou de son sel alcalino-terreux, soit sous forme d'un mélange de l'acide et de son sel alcalino-terreux, soit sous forme de l'acide et de son sel alcalin, soit sous forme d'un mélange du sel alcalin et du sel alcalino-terreux d'un acide carboxylique, soit sous forme d'un mélange de l'acide carboxylique, de son sel alcalin et de son sel alcalino-terreux, et en présence d'hydroxydes de bases alcalino-terreuses et d'eau dans une proportion de 0,01 à 0,1 mol d'hydroxyde de base alcalino-terreuse et de 0,04 à 1 mol d'eau par mol d'aldéhyde isobutyrique.

## Claim

A process for the preparation of 3-hydroxy-2,2,4-trimethylpentyl isobutyrate by reacting isobutyraldehyde in the presence of a basic compound and water, characterized in that isobutyraldehyde is reacted in the presence of from 1.5 to 5 per cent by weight, based on isobutyraldehyde, of a carboxylic acid in the form of the free acid or an alkali metal salt or alkaline earth metal salt thereof, or in the form of a mixture of the acid and an alkaline earth metal salt thereof, or in the form of a mixture of the acid and an alkali metal salt thereof, or in the form of a mixture of the alkali metal salt and the alkaline earth metal salt of a carboyxlic acid, or in the form of a mixture of the carboxylic acid and an alkali metal salt and an alkaline earth metal salt thereof, and in the presence of from 0.01 to 0.1 mole of alkaline earth metal hydroxide and from 0.04 to 1 mole of water per mole of isobutyraldehyde, for a reaction time of from 10 to 300 minutes.